# EUROPÄISCHE PATENTSCHRIFT

(11) **EP 3 107 991 B1**
(45) Veröffentlichungstag und Bekanntmachung des Hinweises auf die Patenterteilung: **02.09.2020**
(21) Anmeldenummer: 15714757.0
(22) Anmeldetag: 13.02.2015
(51) Int. Cl.: C12M 1/107, C02F 3/30, C12M 1/00, C12P 5/02

(54) **VERFAHREN ZUR VORBEREITUNG VON ORGANISCHEN ABFÄLLEN**
METHOD FOR PREPARING ORGANIC WASTE
PROCÉDÉ DE PRÉPARATION DE DÉCHETS ORGANIQUES

(30) Priorität: 20.02.2014 DE 102014102187
(43) Veröffentlichungstag der Anmeldung: 28.12.2016
(73) Patentinhaber: Deger, Oliver, 76182 Karlsruhe (DE)
(72) Erfinder: Deger, Oliver, 76182 Karlsruhe (DE)
(74) Vertreter: Lichti - Patentanwälte Partnerschaft mbB
(86) Internationale Anmeldenummer: PCT/DE2015/000071
(87) Internationale Veröffentlichungsnummer: WO 2015/124136

(56) Entgegenhaltungen:
- EP-A1- 1 118 671
- DE-A1- 3 428 556
- DE-A1- 4 415 017
- DE-A1-102006 032 039

## Beschreibung

Die Erfindung betrifft ein Verfahren zur Vorbereitung von organischen Abfällen, wie Biomasse oder Abwässer, für eine weitere Verarbeitung, insbesondere für eine Vergasung oder Ableitung in bestehende Systeme.

### Stand der Technik

Bei der Zersetzung organischer Substanzen entsteht Gas, und zwar im wesentlichen Umfang CO₂ und Methan. Ein wichtiger Bestandteil dieses Biogases ist Methan, welches als Heizgas und für Verbrennungsantriebe sowie als Ausgangsmaterial für synthetische Produkte, bspw. Acetylen, Synthesegase, HCN und Chlorsubstitutionsprodukte, verwendet werden kann. Auf Grund der Bedeutung des Methans gehen die Bestrebungen dahin, bei der Erzeugung von Biogas einen hohen Methananteil zu erzielen.

Nach dem Stand der Technik werden hierzu ein- oder zweistufige Fermentationsverfahren eingesetzt, bei welchen aus organischen Stoffen mittels anaerober Fermentation Biogas gewonnen wird. Ein großer Bestandteil dieses Gases ist aber auch CO₂ sowie kleinere Anteile von Stickstoff, Schwefelwasserstoff und anderen Komponenten. Diese bekannten Verfahren sind aber bezüglich der erzielten Qualität des erzeugten Biogases und insbesondere der Methanausbeute nicht befriedigend. Dabei ist insbesondere der hohe Schwefel- bzw. Schwefelwasserstoffanteil von etwa 2% unerwünscht, da sich dieser beim Betrieb von Motoren und dem damit verbundenen Einsatz von Katalysatoren störend auswirkt.

Aus diesem Grunde schlägt die EP 1 118 671 A1 ein dreistufiges Verfahren vor, wobei in einem ersten Verfahrensschritt der aeroben Fermentation organsicher Stoff unter aeroben Bedingungen mittels Fermentationsmikroorganismen fermentiert wird, wobei feste und/oder flüssige Rückstände und CO₂-haltige Abgase gebildet und in einem zweiten Verfahrensschritt Rückstände aus dem ersten Verfahrensschritt verschwelt werden, wobei ein Holzkohlenprodukt und Holzgas gebildet werden. In einem dritten Verfahrensschritt der thermophilen Methanfermentation wird Holzgas aus dem zweiten Verfahrensschritt unter anaeroben Bedingungen mittels thermophiler Fermentationsmikroorganismen zu methanhaltigen Biogas fermentiert.

Die DE 10 2006 032 039 A1 beschreibt ein Verfahren zur Herstellung von Methangas aus Abgasen, welche insbesondere Kohlendioxid (CO₂) enthalten und beispielsweise aus Verbrennungsmotoren, Kläranlagen oder Biogasanlagen stammen, indem die Abgase durch einen nach Art eines Gasreformers ausgestalteten Reaktor mit einem porösen Material durchgeleitet werden, welches von einer mit Mikroben versetzten Nährlösung geflutet ist. Als Mikroben gelangen insbesondere Methanosarcina-Stämme oder Bacterium thermoautotrophicum zum Einsatz.

Die EP 1 118 671 A1, welche von einem Stand der Technik ausgeht, bei welchem aus organischen Stoffen Biogas gewonnen wird, indem aus den organischen Stoffen in einem anaeroben Fermenter Methangas erzeugt wird, die Qualität der auf diese Weise erhaltenen Methanausbeute im Hinblick auf (zu) hohe Anteile an Schwefelverbindungen jedoch als unbefriedigend erachtet wird, beschreibt ein Verfahren zum Erzeugen von methanhaltigem Biogas aus organischen Stoffen, indem letztere mittels lebender Mikroorganismen zersetzt und zu Methan umgewandelt werden. Das Verfahren umfasst im Wesentlichen die folgenden drei Verfahrensschritte:
(1) Fermentation der organischen Stoffe in einem aeroben Fermenter unter Einsatz von Fermentationsmikroorganismen, wobei einerseits (feste und/oder flüssige) Rückstände, andererseits CO₂-haltiges Abgas erzeugt wird;
(2) Verschwelung der Rückstände des aeroben Fermenters in einem Verschwelungsreaktor, wobei einerseits ein Holzkohleprodukt, andererseits Holzgas mit Anteilen an vornehmlich Kohlendioxid (CO₂), Kohlenmonoxid (CO), Methan (CH₄) sowie geringen Mengen an Wasserstoff, Stickstoff, Wasser und Ethylen gebildet wird, indem die Rückstände des aeroben Fermenters unter gesteuerter Luftarmut oder Luftabschluss erhitzt bzw. langsam verbrannt werden; und
(3) Überführen des Holzgases des Verschwelungsreaktors an einen thermophilen Methanfermenter, wo es unter anaeroben Bedingungen mittels thermophiler Fermentationsorganismen zu methanhaltigem Biogas fermentiert wird.

Dem Methanogenese-Fermentationsreaktor können dabei auch Rückstände aus dem aeroben Fermenter zusammen mit dem Holzgas aus der Verschwelung direkt zugeführt werden.

### Aufgabe der Erfindung

Der vorliegenden Erfindung liegt vor allem die Aufgabe zugrunde, nochmals das erzeugte Methangas zu verbessern, d.h. ein Biogas zu schaffen, welches über 90% Methan beinhaltet.

### Lösung der Aufgabe

Erfindungsgemäß wird diese Aufgabe mit einem Verfahren der eingangs genannten Art gelöst, wobei
- in einer ersten Verfahrensstufe die organischen Abfälle in einen aeroben Fermenter eingegeben und mit einem Inoculum versetzt werden und
- in einer dritten Verfahrensstufe das in dem aeroben Fermenter entstehende CO₂ einem Gasreformer zugeführt wird, in welchem eine Umwandlung in Methangas erfolgt, wobei ferner
- zwischen den aeroben Fermenter und den Gasreformer ein anaerober Fermenter eingeschaltet ist und Prozesswasser aus dem aeroben Fermenter an den anaeroben Fermenter überführt, mit einem Inoculum versetzt und dort in einer zweiten Verfahrensstufe bis zu Brauchwasser abgebaut wird, wobei das in dem anaeroben Fermenter entstehende CO₂ gleichfalls dem Gasreformer zugeführt wird, in welchem eine Umwandlung in Methangas erfolgt.

Vorteilhafte Ausgestaltungen des erfindungsgemäßen Verfahrens ergeben sich aus den abhängigen Ansprüchen.

Die Erfindung sieht folglich vor, dass eine klare Trennung zwischen dem aeroben und dem anaeroben Fermenter und dem Gasreformer durchgeführt wird. Der Gasreformer ist ausschließlich dazu da, das CO₂ aus den beiden Fermentern zu übernehmen und daraus ein verbessertes Methangas zu machen.

Der aerobe Fermenter, der mit einem entsprechenden aeroben Inoculum geimpft ist, erzeugt durch die Fermentierung zum einen das oben erwähnte CO₂ und zum anderen eine Restbiomasse, die ausgetragen, gepresst, pelletiert und z.B. einem Biomassevergaser zugeführt werden kann. Noch vorhandenes Prozesswasser wird in den anaeroben Fermenter eingeführt und dort weiter verarbeitet. Das CO₂ wird an den Gasreformer überführt.

In dem anaeroben Fermenter befindet sich ein entsprechendes anaerobes Inoculum. In dem anaeroben Fermenter werden vor allem die flüssigen Biomassen zu CO₂ und Methan verarbeitet, wobei letztendlich durch diese Verarbeitung die flüssigen Abwässer in Brauchwasser umgewandelt werden, das in die Kanalisation abgeleitet werden kann. Das entstehende CO₂ und Methan wird dann wiederum dem Gasreformer zugeführt.

Durch spezielle mikrobielle Beschickung auf einer sehr großen Oberfläche erfolgt dann in dem Gasreformer die Aufarbeitung von CO₂ zu einem Methan, wobei ein Methan von über 90% erreicht werden kann.

### Figurenbeschreibung

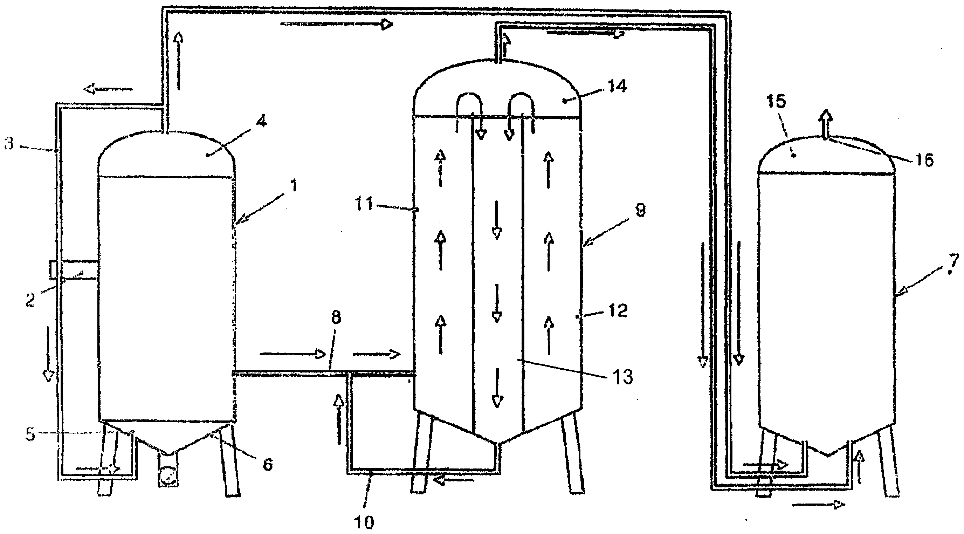

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in ihrer einzigen Figur eine schematische Darstellung einer Anlage zur Ausführung des erfindungsgemäßen Verfahrens zur Vorbereitung von organischen Abfällen für eine weitere Verarbeitung.

Dabei ist mit 1 ein aerober Fermenter bezeichnet, der einen Einlass 2 für Biomasse aufweist. Bevorzugt wird die Masse bis zu einem Feststoffgehalt von 20% in diesen aeroben Fermenter eingebracht und dort mit einem entsprechenden Inoculum beimpft. Bei der Masse handelt es sich infolge des hohen Flüssiganteils um eine flüssige, breiige Masse. Die Prozesstemperatur wird durch eine eingebrachte, nicht näher gezeigte Heizschlange bei konstant 37 bis 38°C eingeregelt. Die Heizschlange steht bevorzugt mit einem Warmwasserkreislauf in Verbindung.

Mit Hilfe von Luft bzw. Sauerstoffdüsen wird die Biomasse zusätzlich umgewälzt.

Die schnell abbaubaren Anteile der Biomasse (Zucker, Fette, Eiweiße) werden unter Luft- und/oder O₂-Zufuhr zu CO₂, CO, H₂ und O₂ reduziert. Das bei Luftzufuhr dabei entstehende Gas hat einen relativ hohen O₂-Gehalt und muss daher einem Sauerstofflimitationsprozess unterzogen werden.

Im oberen Bereich des Fermenters 1 bildet sich ein Gasdom 4, in dem sich dieses Gas ansammelt. Hier erfolgt auch eine ständige Kontrolle des sich sammelnden Gases. Insbesondere bei der Applikation von reinem Sauerstoff sollte das Gas, das noch zu viel Sauerstoff enthält, in einer Schleife 3 von dem Gasdom 4 wieder zurück zu einem Einlass 5 am Boden 6 des Fermenters 1 geführt werden. Dies geschieht solange, bis kein oder nur wenig O₂ im Gasdom 4 vorhanden ist. Dann wird das Gas aus dem Gasdom 4, welches im Wesentlichen CO₂ beinhaltet, an einen Gasreformer 7 überführt.

Nach ca. einem Tag wird die Restbiomasse ausgetragen und z.B. gepresst und pelletiert einem nicht näher gezeigten Biomassevergaser zugeführt. Dort wird es bis auf die mineralischen Bestandteile abgebaut. Das Prozesswasser wird über eine Leitung 8 an einen anaeroben Fermenter 9 überführt und dort bis zu Brauchwasser abgebaut.

Bei dem aeroben Fermenter 1 erfolgt neben einer Temperaturkontrolle auch eine ständige pH-Wert-Bestimmung zur Optimierung des Prozesses.

In den anaeroben Fermenter 9 können neben dem Prozesswasser aus dem aeroben Fermenter 1 auch durch einen weiteren, nicht gezeigten Einlass biologische Abwässer eingeführt werden. Er ist in besonderer Weise dazu bestimmt, diese Abwässer zu reinigen. Hierzu zählen vor allem Abwässer aus der Landwirtschaft und Lebensmittelproduktion, z.B. Gülle, Presswasser, Abwasser von Schlachthöfen, Leimhersteller usw. Erste Versuche haben auch gezeigt, dass der Reaktor zur Reinigung von Deponie-Sickerwässer eingesetzt werden kann.

Eine mikrobielle Beschickung geschieht nach Anzüchtung und Vermehrung eines jeweiligen anaeroben Inoculums durch Besiedlung der im Reaktor befindlichen Trägersysteme. Hierbei werden in unterschiedlicher Dichte Mikrobenbesiedlungsschnüre in dem Reaktor gespannt, die eine großflächige Ansiedlungsmöglichkeit bieten. Diese können aus Plastikschnüren, Hanfseilen oder aus Bahnen von reinen Kohlenstoffgeweben bestehen. Vor allem das letztere Material hat eine sehr große Oberfläche von bis zu 1800 qm/g als Träger für die Bakterien.

Der Flüssigkeitstransport im Reaktor erfolgt auch über eine Außenleitung 10. Die Flüssigkeit steigt in zwei äußeren Kammern 11 und 12 auf und wird dann durch ein Zentralrohr 13 wieder nach unten geführt. Dieser Kreislauf wird bis zu einem ausreichenden Abbau zirkulär geführt und danach die Restmasse ausgetragen, gepresst bzw. pelletiert. Die Flüssigkeit kann als Brauchwasser in die Kanalisation verbracht werden.

Der anaeroben Fermenter 9 baut verflüssigte Biomasse bzw. Wasser bis zu einem Feststoffanteil von ca. 10% in einem Zeitraum von maximal 2 bis 3 Tagen ab.

In einem Gasdom 14 sammelt sich CO₂ und Methan an, wobei ein Methangehalt von 60 bis 82% in nicht unerheblicher Menge erreicht wird. H₂S wird dagegen auf unter 0,1 ppm reduziert.

Die Temperatursteuerung von 55 bis 56°C erfolgt bevorzugt durch eine Heizspirale, die ebenfalls von außen mit heißem Wasser gesteuert wird. Neben einer ständigen Temperaturkontrolle werden auch permanente pH-Messungen und CH₄-Kontrollen durchgeführt.

Der Gasreformer 7 zeichnet sich durch seine besondere Fähigkeit aus, CO₂ in einem Hochtemperatur-Methanogenese-Prozess in CH₄ umzubauen. Dazu benötigt er vor allem eine möglichst große Oberfläche als Trägersubstanz für die thermophilen Mikroben. Die mikrobielle Beschickung erfolgt durch einen Methanolsarcinastamm und einem Bakterium Thermoautotrophikum auf einem speziellen Braunkohle-Industriekoks mit großer Oberfläche. Innerhalb des Reformers befinden sich ca. 500 kg Industriekoks. Ein Gramm von diesem Industriekoks hat eine Oberfläche von 220 qm, so dass die Koksfüllung somit ungefähr einer Oberfläche von 110 qkm bzw. 110.000.000 qm bietet.

Im Reaktor befindet sich dieser Industriekoks oberhalb einer Nährlösung für die Mikroben und dem Wasser, das sich bei der Reaktion bildet, getrennt durch einen Gitterrost.

Eine Temperatursteuerung von 75 bis 78°C wird durch eine Heizspirale, die in dem Gasreformer 7 eingebracht ist, von außen durch Heißwasser gesteuert. Neben der mehrfachen Temperaturkontrolle wird eine permanente Messung von CO₂, CO sowie CH₄ durchgeführt, ebenso wird der PH-Wert zur Steuerung des optimalen Prozesses kontrolliert.

Bei höheren CO₂ Anteilen kann das Gas aus dem Gasdom 15 im Kreislauf zurückgeführt werden, bis der gewünschte CH₄-Gehalt (z.B. Gehalt von über 90%) erreicht ist.

Dieser Gasreformer 7 ist in besonderer Weise dazu geeignet, CO₂-Abgase in CH₄ umzuwandeln, um eine CO₂-Emission zu ersetzen. Das ist in besonderer Weise wichtig unter anderem für Holzvergaser, Holz- und Kohlekraftwerke und Großmotoren, die ihr Gas aus mesophilen Methanogeneseprozessen (niedriger CH₄-Gehalt) erhalten. Jedoch können mit einem solchem Gasreformer 7 auch alle herkömmlichen Kläranlagen bzw. Biogasanlagen nachgerüstet werden.

Das Methangas mit einem Methangehalt von 80 bis 95% Methan wird aus einem Auslass 16 einer weiteren Verwertung zugeführt.

### Bezugszeichenliste

- 1: - aerober Fermenter
- 2: - Einlass
- 3: - Schleife
- 4: - Gasdom
- 5: - Einlass
- 6: - Boden
- 7: - Gasreformer
- 8: - Leitung
- 9: - anaerober Fermenter
- 10: - Außenleitung
- 11: - Außenkammer
- 12: - Außenkammer
- 13: - Zentralrohr
- 14: - Gasdom
- 15: - Gasdom
- 16: - Auslass

## Patentansprüche

1. Verfahren zur Vorbereitung von organischen Abfällen, wie Biomassen oder Abwässern, für eine weitere Verarbeitung, insbesondere für eine Vergasung, wobei
- in einer ersten Verfahrensstufe die organischen Abfälle in einen aeroben Fermenter (1) eingegeben und mit einem Inoculum versetzt werden und
- in einer dritten Verfahrensstufe das in dem aeroben Fermenter (1) entstehende CO₂ einem Gasreformer (7) zugeführt wird, in welchem eine Umwandlung in Methangas erfolgt, wobei ferner
- zwischen den aeroben Fermenter (1) und den Gasreformer (7) ein anaerober Fermenter (9) eingeschaltet ist und Prozesswasser aus dem aeroben Fermenter (1) an den anaeroben Fermenter (9) überführt, mit einem Inoculum versetzt und dort in einer zweiten Verfahrensstufe bis zu Brauchwasser abgebaut wird, wobei das in dem anaeroben Fermenter (9) entstehende CO₂ gleichfalls dem Gasreformer (7) zugeführt wird, in welchem eine Umwandlung in Methangas erfolgt.

2. Verfahren nach Anspruch 1, **dadurch gekennzeichnet, dass** die Biomasse in den aeroben Fermenter (1) als flüssige, breiige Masse eingegeben wird.

3. Verfahren nach Anspruch 1 oder 2, **dadurch gekennzeichnet, dass** die Temperatur in dem aeroben Fermenter (1) bei 37°C bis 38°C gehalten wird.

4. Verfahren nach wenigstens einem der vorhergehenden Ansprüche, **dadurch gekennzeichnet, dass** dem aeroben Fermenter (1) Sauerstoff zugeführt wird.

5. Verfahren nach wenigstens einem der vorhergehenden Ansprüche, **dadurch gekennzeichnet, dass** das in dem aeroben Fermenter (1) entstehende Gas bezüglich des Sauerstoffanteils limitiert wird.

6. Verfahren nach wenigstens einem der vorhergehenden Ansprüche, **dadurch gekennzeichnet, dass** das in einem Gasdom (4) des aeroben Fermenters (1) sich ansammelnde CO₂ erst dann an den Gasreformer (7) weitergeleitet wird, wenn der in dem Gasdom (4) ebenfalls vorhandene Sauerstoff auf ein Minimum reduziert ist, wobei das Gas, welches sich im Gasdom (4) ansammelt, insbesondere so lange im Kreislauf geführt wird, bis der Sauerstoffgehalt auf ein Minimum reduziert ist bzw. kein Sauerstoff mehr vorhanden ist.

7. Verfahren nach einem der vorhergehenden Ansprüche, **dadurch gekennzeichnet, dass** Rest-Biomasse aus dem aeroben Fermenter (1) ausgetragen und weiterverarbeitet wird.

8. Verfahren nach wenigstens einem der vorhergehenden Ansprüche, **dadurch gekennzeichnet, dass** die Umwandlung des CO₂ in dem Gasreformer (7) in Methan auf mikrobiellem Wege geschieht, wobei insbesondere eine mikrobielle Beschickung des Gasreformers (7) durch einen Methanosarcinastamm und einem Bakterium Thermoautotrophicum auf einem Braunkohle-Industriekoks mit großer Oberfläche erfolgt.

9. Verfahren nach wenigstens einem der vorhergehenden Ansprüche, **dadurch gekennzeichnet, dass** in dem Gasreformer (7) eine Temperatur von 75°C bis 78°C gehalten wird.

10. Verfahren nach einem der Ansprüche 1 bis 9, **dadurch gekennzeichnet, dass** in dem anaeroben Fermenter (9) eine Temperatur von 55°C bis 56°C gehalten wird.

11. Verfahren nach einem der Ansprüche 1 bis 10, **dadurch gekennzeichnet, dass** der anaerobe Fermenter (9) mit Abwässern beschickt wird.

12. Verfahren nach Anspruch 11, **dadurch gekennzeichnet, dass** der anaerobe Fermenter (9) mit Abwässern aus der Landwirtschaft und aus der Lebensmittelproduktion, z.B. Gülle, Presswasser, Abwasser von Schlachthöfen, Leimhersteller und Deponie-Sickerwässer, beschickt wird.

13. Verfahren nach wenigstens einem der Ansprüche 1 bis 12, **dadurch gekennzeichnet, dass** sich in einem Gasdom (14) des anaeroben Fermenters (9) CO₂ und Methan ansammelt, welches zu dem nachfolgenden Gasreformer (7) geführt wird.

14. Verfahren nach wenigstens einem der Ansprüche 1 bis 13, **dadurch gekennzeichnet, dass** Gas aus dem aeroben Fermenter (1) auch in den anaeroben Fermenter (9) überführt wird.

15. Verfahren nach Anspruch 13 oder 14, **dadurch gekennzeichnet, dass** das sich im Gasdom (14) des anaeroben Fermenter (9) ansammelnde Gas zumindest teilweise im Kreislauf geführt wird, bis im Gasdom (14) nur CO₂ und Methan vorhanden ist.

## Claims

1. Method of preparing organic wastes, such as biomass or wastewater, for further processing, especially for gasification, wherein
- in a first process stage the organic wastes are introduced into an aerobic fermenter (1) and admixed with an inoculant and
- in a third process stage the CO₂ formed in the aerobic fermenter (1) is supplied to a gas reformer (7) in which there is a conversion to methane gas, wherein
- connected between the aerobic fermenter (1) and the gas reformer (7) there is also an anaerobic fermenter (9), and process water is transferred from the aerobic fermenter (1) to the anaerobic fermenter (9), is admixed with an inoculum and is degraded in a second process stage to give service water, wherein the CO₂ formed in the anaerobic fermenter (9) is likewise supplied to the gas reformer (7) in which there is a conversion to methane gas.

2. Method according to Claim 1, **characterized in that** the biomass is introduced into the aerobic fermenter (1) in the form of a liquid slurrylike mass.

3. Method according to Claim 1 or 2, **characterized in that** the temperature in the aerobic fermenter (1) is kept at 37°C to 38°C.

4. Method according to at least one of the preceding claims, **characterized in that** the aerobic fermenter (1) is supplied with oxygen.

5. Method according to at least one of the preceding claims, **characterized in that** the gas formed in the aerobic fermenter (1) is limited in terms of its oxygen content.

6. Method according to at least one of the preceding claims, **characterized in that** the CO₂ that collects in a gas dome (4) of the aerobic fermenter (1) is passed onward to the gas reformer (7) only when the oxygen likewise present in the gas dome (4) is reduced to a minimum, with circulation of the gas that collects in the gas dome (4) especially until the oxygen content has been reduced to a minimum or there is no oxygen present any longer.

7. Method according to any of the preceding claims, **characterized in that** residual biomass from the aerobic fermenter (1) is discharged and processed further.

8. Method according to at least one of the preceding claims, **characterized in that** the conversion of the CO₂ in the gas reformer (7) to methane is accomplished by a microbial route, especially by means of a microbial charge of the gas reformer (7) with a Methanosarcina strain and a Thermoautotrophicum bacterium on a brown coal industrial coke of high surface area.

9. Method according to at least one of the preceding claims, **characterized in that** a temperature of 75°C to 78°C is maintained in the gas reformer (7).

10. Method according to any of Claims 1 to 9, **characterized in that** a temperature of 55°C to 56°C is maintained in the anaerobic fermenter (9).

11. Method according to any of Claims 1 to 10, **characterized in that** the anaerobic fermenter (9) is charged with wastewater.

12. Method according to Claim 11, **characterized in that** the anaerobic fermenter (9) is charged with wastewater from agriculture and from food production, for example liquid manure, press water, wastewater from slaughterhouses and glue manufacturers, and landfill leachate.

13. Method according to at least one of Claims 1 to 12, **characterized in that** CO₂ and methane collects in a gas dome (14) of the anaerobic fermenter (9) and is routed to the downstream gas reformer (7).

14. Method according to at least one of Claims 1 to 13, **characterized in that** gas from the aerobic fermenter (1) is also transferred into the anaerobic fermenter (9).

15. Method according to Claim 13 or 14, **characterized in that** the gas that collects in the gas dome (14) of the anaerobic fermenter (9) is at least partly circulated until only CO₂ and methane is present in the gas dome (14).

## Revendications

1. Procédé de préparation de déchets organiques, tels que de la biomasse ou des eaux usées, pour un traitement supplémentaire, notamment pour une gazéification, dans lequel :
- lors d'une première étape de procédé, les déchets organiques sont admis dans un fermenteur aérobie (1) et ajoutés à un inoculant ; et
- lors d'une troisième étape de procédé, le CO₂ produit dans le fermenteur aérobie (1) est amené à un reformeur de gaz (7) dans lequel une transformation en méthane se produit, dans lequel en outre :
- un fermenteur anaérobie (9) est inséré entre le fermenteur aérobie (1) et le reformeur de gaz (7) et dans lequel les eaux de traitement provenant du fermenteur aérobie (1) sont transférées au fermenteur anaérobie (9), ajoutées à un inoculant et décomposées à cet endroit, lors d'une deuxième étape de procédé, en eau industrielle, le CO₂ produit dans le fermenteur anaérobie (9) étant également amené au reformeur de gaz (7) dans lequel une transformation en méthane se produit.

2. Procédé selon la revendication 1, **caractérisé en ce que** la biomasse est entrée dans le fermenteur aérobie (1) sous forme de masse fluide pultacée.

3. Procédé selon la revendication 1 ou 2, **caractérisé en ce que** la température dans le fermenteur aérobie (1) est maintenue à 37°C à 38°C.

4. Procédé selon au moins l'une quelconque des revendications précédentes, **caractérisé en ce que** de l'oxygène est amené au fermenteur aérobie (1).

5. Procédé selon au moins l'une quelconque des revendications précédentes, **caractérisé en ce que** le gaz produit dans le fermenteur aérobie (1) est limité en termes de teneur en oxygène.

6. Procédé selon au moins l'une quelconque des revendications précédentes, **caractérisé en ce que** le CO₂ s'accumulant dans un dôme de gaz (4) du fermenteur aérobie (1) n'est transféré au reformeur de gaz (7) que lorsque l'oxygène également présent dans le dôme de gaz (4) est réduit à un minimum, le gaz s'accumulant dans le dôme de gaz (4), n'étant notamment amené dans le circuit que lorsque la teneur en oxygène est réduite à un minimum et/ou qu'il n'y a plus d'oxygène présent.

7. Procédé selon l'une quelconque des revendications précédentes, **caractérisé en ce que** la biomasse restante est sortie du fermenteur aérobie (1) et retraitée.

8. Procédé selon au moins l'une quelconque des revendications précédentes, **caractérisé en ce que** la transformation du CO₂ en méthane dans le reformeur de gaz (7) se produit par voies microbiennes, un chargement microbien du reformeur de gaz (7) se produisant notamment par le biais d'une souche Methanosarcina et d'une bactérie Thermoautotrophicum placées sur un coke industriel à base de lignite de grande surface.

9. Procédé selon au moins l'une quelconque des revendications précédentes, **caractérisé en ce qu'**une température de 75°C à 78°C est maintenue dans le reformeur de gaz (7).

10. Procédé selon l'une quelconque des revendications 1 à 9, **caractérisé en ce qu'**une température de 55°C à 56°C est maintenue dans le fermenteur anaérobie (9).

11. Procédé selon l'une quelconque des revendications 1 à 10, **caractérisé en ce que** le fermenteur anaérobie (9) est alimenté en eaux usées.

12. Procédé selon la revendication 11, **caractérisé en ce que** le fermenteur anaérobie (9) est chargé en eaux usées d'origine agricole et alimentaire, par exemple du lisier, de l'eau de pressage, des eaux usées d'abattoirs, de fabricants de colle et des eaux de suintement de décharge.

13. Procédé selon au moins l'une quelconque des revendications 1 à 12, **caractérisé en ce que** du CO₂ et du méthane s'accumulent dans un dôme de gaz (14) du fermenteur anaérobie (9) et que le méthane est amené au reformeur de gaz (7) suivant.

14. Procédé selon au moins l'une quelconque des revendications 1 à 13, **caractérisé en ce que** le gaz provenant du fermenteur aérobie (1) est également transféré dans le fermenteur anaérobie (9).

15. Procédé selon la revendication 13 ou 14, **caractérisé en ce que** le gaz s'accumulant dans le dôme de gaz (14) du fermenteur anaérobie (9) est amené au moins en partie dans le circuit jusqu'à ce que seuls du CO₂ et du méthane soient présents dans le dôme de gaz (14).
